# EUROPÄISCHE PATENTANMELDUNG

(11) **EP 1 097 692 A2**
(43) Veröffentlichungstag der Anmeldung: **09.05.2001**
(21) Anmeldenummer: 00124087.8
(22) Anmeldetag: 06.11.2000
(51) Int. Cl.: A61K 6/02, A61K 6/00

(54) **Materialkombination für Zahnersatz**

(30) Priorität: 04.11.1999 DE 19953180
(71) Anmelder: Aichhorn, Wilfried, 83278 Traunstein (DE)
(72) Erfinder: Aichhorn, Wilfried, 83278 Traunstein (DE)
(74) Vertreter: Viering, Jentschura & Partner

(57) **Zusammenfassung**

Materialkombination für Zahnersatz, die ein Zahnersatzmaterial als eine erste Komponente und ein selbsthärtendes klebriges Bindemittel, das aus der Frucht der Stemadenia-Apogynazea-Pflanze gewonnen ist, als eine zweite Komponente zum Verbinden der ersten Komponente mit der verbliebenen Zahnsubstanz aufweist. Die Materialkombination für Zahnersatz beruht auf biologischer Basis und zeichnet sich durch seine hervorragende Verträglichkeit und dauerhafte Haltbarkeit aus. Die erste Komponente kann für Zahnfüllungen aus einem Gemisch aus einem Basismaterial, insbesondere gemahlenem Muschelkalk, und dem Stemadenia-Apogynazea-Pflanzensaft bestehen. Als erste Komponente eignen sich aber auch Inlays, Brücken und Kronen aus dem Samen der Steinnußpalme oder der Schale der Austernmuschel.

## Beschreibung

Die Erfindung betrifft eine Materialkombination für Zahnersatz.

Die derzeit bekannten Zahnersatzmaterialien, insbesondere für Zahnfüllungen, Inlays, Brücken, Kronen oder dergleichen bestehen meist auf metallischer Basis, die aufgrund ihrer oft toxischen Wirkung von vielen Benutzern nicht vertragen werden. Zwar weisen diese metallischen Zahnersatzmaterialien eine hohe Festigkeit und damit eine gute Haltbarkeit auf, jedoch können diese Materialien aufgrund von entstehenden Nebenwirkungen nicht umfassend eingesetzt werden.

Zum Verschließen von Foramen in den Wurzeln toter Zähne gibt es bereits gut verträgliches Material. Da dieses Material jedoch sehr weich ist, ist es nur geeignet, Hohlräume zu verschließen, die keiner Belastung standhalten müssen.

Aufgabe der Erfindung ist es, eine Materialkombination für Zahnersatz zu schaffen, die als Ersatz für verlorene Zahnsubstanz geeignet ist, im Mund gut haltbar und gut verträglich ist.

Diese Aufgabe wird gelöst mit einer Materialkombination für Zahnersatz aus einem Zahnersatzmaterial als einer ersten Komponente und einem selbsthärtenden klebrigen Bindemittel, das aus der Frucht der Stemadenia-Apogynazea-Pflanze gewonnen ist, als einer zweiten Komponente zum Verbinden der ersten Komponente mit der verbliebenen Zahnsubstanz.

Dabei ist vorzugsweise auch das Zahnersatzmaterial als eine erste Komponente wie das selbsthärtende klebrige Bindemittel als der zweiten Komponente aus biologischem Material. Das selbsthärtende klebrige Bindemittel ist der aus der Frucht der Stemadenia-Apogynazea-Pflanze gewonnene Saft, der eine klebrige Masse bildet, die sich sowohl mit dem Zahnersatzmaterial als auch mit der verbliebenen Zahnsubstanz ausgezeichnet verbindet, wodurch eine dauerhafte Haltbarkeit des Zahnersatzes sichergestellt ist.

Vorzugsweise ist das Zahnersatzmaterial ein Gemisch aus einem Basismaterial und dem selbsthärtenden klebrigen Bindemittel, das aus der Frucht der Stemadenia-Apogynazea-Pflanze gewonnen wird. Von diesem Bindemittel wird das Basismaterial zusammengehalten und fest mit der verbliebenen Zahnsubstanz verkittet. Dieses erfindungsgemäße Gemisch, das für Zahnfüllungen vorgesehen ist, schrumpft bei der Anwendung nicht, ist sehr fest, hält den im Zahnbereich üblichen Belastungen stand und ist gegen Temperaturschwankungen unempfindlich.

Vorzugsweise ist das Basismaterial ein von Schwermetallen freier pulverisierter Stoff der mit dem Bindemittel, nämlich dem aus der Frucht der Stemadenia-Apogynazea-Pflanze gewonnenen Saft, gemischt wird und eine zum Füllen von Zähnen geeignete Masse ergibt. Der aus der Frucht der Stemadenia-Apogynazea-Pflanze gewonnene Saft bindet einerseits das Basismaterial und verfestigt andererseits das so entstandene Gemisch durch seine selbsthärtende Eigenschaft im Zahn. Nach dem Einbringen des als Zahnfüllung ausgebildeten Zahnersatzmaterials in den Zahn sind keine zusätzlichen Vorkehrungen notwendig, das Material wird von selbst fest.

Vorzugsweise ist der als Basismaterial beigemischte Stoff gemahlener Muschelkalk. Bevorzugt wird dieser Muschelkalk vor dem Beimischen hinsichtlich seiner Reinheit, daß heißt auf das Nicht-Vorhandensein von Schwermetallbestandteilen überprüft. Die Pflanzen-Komponente sowie der Muschelkalk sind reine Naturprodukte. Dieses Zahnfüllungsmaterial beruht vollkommen auf biologischer Basis und zeichnet sich somit durch seine hervorragende Verträglichkeit bei den Benutzern eines derartigen Zahnsubstanz-Ersatzes aus.

Das biologische Material muß so verarbeitet und aufbereitet werden, daß man es zum Füllen von Zähnen verwenden kann. Dazu ist es nötig, den aus der Pflanzen-Frucht gewonnenen Stoff mit gemahlenen Muschelkalk, der vorher auf seine Reinheit überprüft wurde, zu vermischen, um eine stabilere Masse zu erhalten, die dann im Zahn selbständig aushärtet. Da die Frucht in einer bestimmten Reifephase die idealen Eigenschaften besitzt, muß die Frucht in diesem Stadium haltbar gemacht werden. Dies kann durch Lagerung in Behältern geschehen, die mit Helium geflutet wurden und dann unter 2 bar Druck gehalten werden. Die Frucht ist vorerst in Mexico, Guatemala und Belice gefunden worden. Die fertig gemischte Masse ist selbsthärtend, verbindet sich gut mit der Zahnsubstanz und ist ein Naturprodukt.

Nach einer anderen bevorzugten Ausgestaltung der erfindungsgemäßen Materialkombination ist das Zahnersatzmaterial als der ersten Komponente aus dem Samen der Steinnußpalme, der sogenannten Steinuß. Der Zahnersatz wird im Kopierfräsverfahren aus dem Samenkörper hergestellt und kann beispielsweise als Inlay, Krone oder Brücke ausgebildet sein. Das Steinnuß-Material verbindet sich hervorragend mit dem selbsthärtenden klebrigen Bindemittel, das aus der Frucht der Stemadenia Apogynazea-Pflanze gewonnen wird, so daß sich der Pflanzen-Saft bestens zum Ein- bzw. Ankleben des Zahnersatzes in bzw. an die verbliebene Zahnsubstanz eignet. Somit beruht auch diese Ausführungsform auf rein biologischer Basis. Der Samen der Steinnußpalme liefert mit seinem sehr harten, aus Reservecellulose bestehenden Nährgewebe vegetatibles Elfenbein. Das Steinnuß-Material kann auch ohne Verwendung des aus der Frucht der Stemadenia Apogynazea-Pflanze gewonnenen Bindemittels mit einem anderen geeigneten Zementierungsmittel als Zahnersatz verwendet werden.

Nach noch einer anderen Ausgestaltung der Erfindung kann der Zahnersatz als erste Komponente aus der Schale der Austernmuschel (Perlmutt) bestehen und im Kopierfräsverfahren aus dieser hergestellt sein. Die Schale der Austernmuschel verbindet sich ebenfalls hervorragend mit dem selbsthärtenden Bindemittel, kann aber auch ohne Verwendung desselben als Zahnersatz verwendet werden.

Die Pflanze Stemadenia-Apogynazea bringt in ihren Früchten einen Satt hervor, der sehr klebrig ist. Mit dieser weißen Milch haben die Mayas die Fugen ihrer Pyramiden verschlossen. Noch heute nach 1500 Jahren steht man staunend vor diesen Bauwerken und sucht vergebens nach einem Riß in der Verbindung zweier Steine. Die Verbindung ist sehr fest, weist keinerlei Schrumpfungen auf und halt Temperaturschwankungen wunderbar stand.

Der aus der Materialkombination hergestellte Zahnersatz besteht vorzugsweise ausschließlich aus Naturprodukten, die im Mund ohne schädliche Nebenwirkungen bei sehr langer Lebensdauer verträglich sind.

## Patentansprüche

1. Materialkombination für Zahnersatz aus einem Zahnersatzmaterial als einer ersten Komponente und einem selbsthärtenden klebrigen Bindemittel, das aus der Frucht der Stemadenia-Apogynazea-Pflanze gewonnen ist, als einer zweiten Komponente zum Verbinden der ersten Komponente mit der verbliebenen Zahnsubstanz.

2. Materialkombination nach Anspruch 1, bei der die erste Komponente aus einem Gemisch aus einem Basismaterial und dem selbsthärtenden klebrigen Bindemittel gebildet ist.

3. Materialkombination nach Anspruch 2, bei der das Basismaterial gemahlener Muschelkalk ist.

4. Materialkombination nach Anspruch 1, bei der die erste Komponente im Kopierfräsverfahren aus dem Samen der Steinnußpalme (Steinnuß) hergestellt ist.

5. Materialkombination nach Anspruch 1, bei der die erste Komponente im Kopierfräsverfahren aus der Schale der Austernmuschel hergestellt ist.
